(19) Europäisches Patentamt
European Patent Office
Office européen des brevets

(11) Numéro de publication: **0 520 016 B1**

(12) # FASCICULE DE BREVET EUROPEEN

(45) Date de publication de fascicule du brevet: **05.10.94**

(51) Int. Cl.⁵: **C07C 275/28**, C07C 273/18, A61K 31/17

(21) Numéro de dépôt: **91906784.3**

(22) Date de dépôt: **11.03.91**

(86) Numéro de dépôt internationale : **PCT/FR91/00195**

(87) Numéro de publication internationale : **WO 91/13862 (19.09.91 91/22)**

(54) **DERIVES DE L'UREE, LEUR PREPARATION ET LES MEDICAMENTS LES CONTENANT.**

(30) Priorité: **13.03.90 FR 9003187**

(43) Date de publication de la demande: **30.12.92 Bulletin 92/53**

(45) Mention de la délivrance du brevet: **05.10.94 Bulletin 94/40**

(84) Etats contractants désignés: **AT BE CH DE DK ES FR GB GR IT LI LU NL SE**

(56) Documents cités: **EP-A- 0 397 556**

(73) Titulaire: **RHONE-POULENC RORER S.A.**
**20, avenue Raymond Aron**
**F-92165 Antony Cédex (FR)**

(72) Inventeur: **BOURZAT, Jean-Dominique**
**20, avenue de la Libération**
**F-94300 Vincennes (FR)**
Inventeur: **CAPET, Marc**
**10, rue de la Galaise**
**F-94320 Thiais (FR)**
Inventeur: **COTREL, Claude**
**17, avenue du Dr.-Arnold-Netter**
**F-75012 Paris (FR)**
Inventeur: **GUYON, Claude**
**17 bis, avenue Henri-Martin**
**F-94100 Saint-Maur-des-Fosses (FR)**
Inventeur: **MANFRE, Franco**
**40, rue Clément-Perrot**
**F-94400 Vitry-sur-Seine (FR)**
Inventeur: **ROUSSEL, Gérard**
**20 ter, rue des Carrières**
**F-91450 Soisy-sur-Seine (FR)**

(74) Mandataire: **Pilard, Jacques et al**
**RHONE-POULENC RORER S.A.**
**Direction Brevets (t144)**
**20 avenue Raymond Aron**
**F-92165 Antony Cédex (FR)**

EP 0 520 016 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des composés de formule :

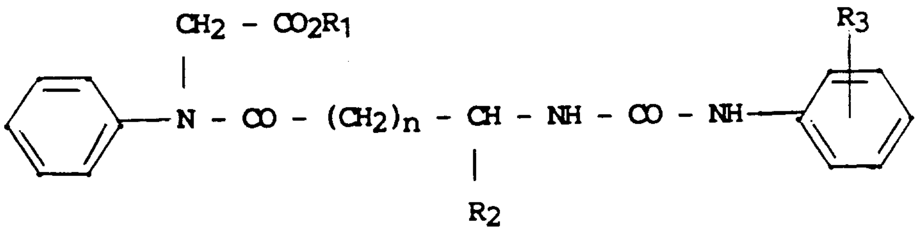

(I)

leur préparation et les médicaments les contenant.

Dans la formule (I) :

- $R_1$ représente un radical alkyle,
- $R_2$ représente un radical phényle ou une chaîne $-(CH_2)_m-CO-R_4$ dans laquelle m est égal à 0, 1 ou 2 et $R_4$ représente un radical hydroxy, alcoxy ou amino,
- $R_3$ représente un atome d'hydrogène ou d'halogène ou un radical alkyle, alcoxy ou alkylthio,
- n est égal à 0 ou 1.

Dans les définitions qui précèdent et celles qui seront citées ci-après, sauf mention contraire, les radicaux alkyle et alcoxy contiennent 1 à 4 atomes de carbone en chaîne droite ou ramifiée.

Dans la formule (I), les atomes d'halogène sont de préférence des atomes de chlore, de brome ou de fluor.

Les racémiques et les énantiomères des composés de formule (I) font partie de l'invention.

Les composés de formule (I), pour lesquels $R_2$ représente un radical phényle ou une chaîne $-(CH_2)_m-CO-R_4$ dans laquelle m est égal à 0, 1 ou 2 et $R_4$ représente un radical alcoxy, peuvent être obtenus par action d'une amine de formule :

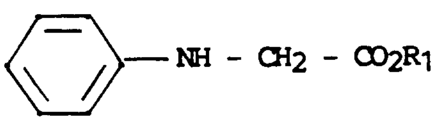

(II)

dans laquelle $R_1$ a les mêmes significations que dans la formule (I), sur un acide de formule :

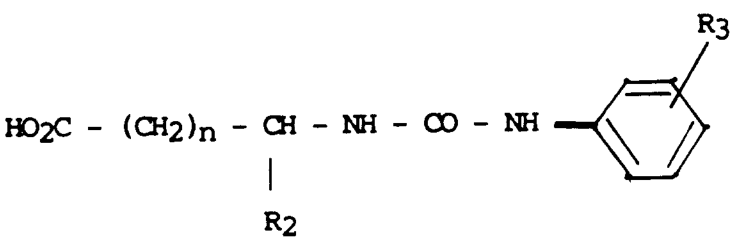

(III)

dans laquelle n et $R_3$ ont les mêmes significations que dans la formule (I) et $R_2$ a les mêmes significations que précédemment, ou un dérivé réactif de cet acide.

Lorsque l'on met en oeuvre l'acide, on opère en présence d'un agent de condensation peptidique tel qu'un carbodiimide (par exemple le dicyclohexylcarbodiimide) ou le N,N'-diimidazolecarbonyle dans un solvant inerte tel qu'un éther (par exemple THF, dioxanne), un amide (par exemple DMF) ou un solvant chloré (par exemple chlorure de méthylène, dichloro-1,2 éthane, chloroforme) à une température comprise entre 0 °C et la température de reflux du mélange réactionnel.

Lorsque l'on met en oeuvre un dérivé réactif de l'acide, il est possible de faire réagir l'anhydride, un anhydride mixte, un halogénure d'acide ou un ester (qui peut être choisi parmi les esters activés ou non de l'acide).

On opère alors soit en milieu organique, éventuellement en présence d'un accepteur d'acide tel qu'une base organique azotée (par exemple une trialkylamine, une pyridine, le diaza-1,8 bicyclo[5.4.0] undécène-7 ou le diaza-1,5 bicyclo[4.3.0] nonène-5), dans un solvant tel que cité ci-dessus, ou un mélange de ces solvants, à une température comprise entre 0 ° C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40 ° C.

Les amines de formule (II) peuvent être obtenues par action d'aniline sur un dérivé de formule :

$Hal - CH_2 - CO_2R_1$ (IV)

dans laquelle $R_1$ a les mêmes significations que dans la formule (I) et Hal représente un atome d'halogène (de préférence chlore ou brome).

Cette réaction s'effectue de préférence au sein d'un solvant inerte tel que l'acétontrile, le diméthylformamide, le tétrahydrofuranne, à la température d'ébullition du solvant.

Les dérivés de formule (IV) peuvent être obtenus par application ou adaptation des méthodes décrites dans le Beilstein 2,213 et 2,197.

Les acides de formule (III) peuvent être préparés par action d'un phénylisocyanate de formule :

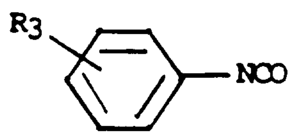

(V)

dans laquelle $R_3$ a les mêmes significations que dans la formule (I), sur un dérivè de formule :

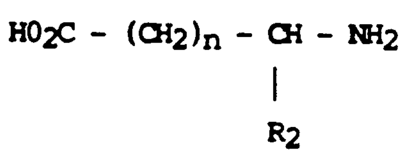

(VI)

dans laquelle n a les mêmes significations que dans la formule (I) et $R_2$ représente un radical phényle ou une chaîne $-(CH_2)_m-CO-R_4$ dans laquelle m est égal à 0, 1 ou 2 et $R_4$ représente un radical alcoxy.

Cette réaction s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10 ° C et la température d'ébullition du solvant.

Les isocyantes de formule (V) peuvent être obtenus par application ou adaptation de la méthode décrite par R. RICHTER et coll., The Chemistry of Cyanate and their thio derivatives, S. PATAI, part 2, Wiley New York (1977).

Les dérivés de formule (VI) pour lesquels $R_2$ représente une chaîne $-(CH_2)_m-CO-R_4$ dans laquelle $R_4$ représente un radical alcoxy peuvent être obtenus par application ou adaptation de la méthode décrite par D. COLEMAN, J. Chem. Soc., 2294 (1951).

Les composés de formule (I) pour lesquels $R_2$ représente une chaîne $-(CH_2)_m-CO-R_4$ dans laquelle $R_4$ représente un radical hydroxy peuvent être préparés par hydrolyse du composé correspondant de formule (I) pour lequel $R_4$ représente un radical alcoxy.

Cette hydrolyse s'effectue généralement au moyen d'une base telle que la soude ou la potasse, dans un solvant inerte tel que l'eau, un alcool ou un mélange de ces solvants, à une température voisine de 20 ° C suivie de l'action d'un acide pour libérer le produit sous forme acide.

Les composés de formule (I) pour lesquels $R_2$ représente une chaîne $-(CH_2)_m-CO-R_4$ dans laquelle $R_4$ représente un radical amino peuvent être obtenus par action d'ammoniac sur le composé correspondant pour lequel $R_4$ représente un radical alcoxy.

Cette réaction s'effectue généralement au sein d'un alcool tel que le méthanol, l'éthanol, à une température voisine de 25 ° C.

Les énantiomères des composés de formule (I) peuvent être obtenus par dédoublement des racétamiques par exemple par chromatographie sur colonne chirale selon W.H. PIRCKLE et coll., asymetric synthesis, vol. 1, Academic Press (1983) ou par synthèse à partir des précurseurs chiraux.

Les composés de formule (I) peuvent être purifiés par les méthodes connues habituelles, par exemple, par cristallisation, chromatographie, extraction...

Les composés de formule (I) pour lesquels $R_4$ représente un radicalhydroxy, peuvent être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac ou d'une amine sur un composé de formule (I), dans un solvant tel qu'un alcool, un éther ou l'eau ou par réaction d'échange avec sel d'un acide organique. Le sel formé est séparé par les méthodes habituelles.

Ces sels font également partie de l'invention.

Comme exemples de sels pharmaceutiquement acceptables, peuvent être cités les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-$\beta$-phénéthylamine, choline, arginine, leucine, lysine, N-méthylglucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et le prévention des désordres liés à la CCK et à la gastrine au niveau du système nerveux et de l'appareil gastrointestinal.

C'est ainsi que ces composés peuvent être utilisés pour le traitement ou la prévention de psychoses, des trouble anxieux, de la maladie de Parkinson, de la diskinésie tardive, du syndrome du colon irritable, de la pancréatite aiguë, des ulcères, des désordres de la motilité intestinale, de certaines tumeurs de l'oesophage inférieur, du colon et de l'intestin, et comme régulateur de l'appétit.

Ces composés ont également un effet de potentialisation sur l'activité analgésique des médicaments narcotiques et non narcotiques.

L'affinité des composés de formule (I) pour les récepteurs de la CCK a été déterminée selon une technique inspirée de celle de A. SAITO et coll., J. Neuro. Chem., 37, 483-490 (1981) au niveau du cortex cérébral et au niveau du pancréas.

Dans ces tests, la $CI_{50}$ des composés de formule (I) est généralement égale ou inférieure à 1000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 16-23 (1989), REYFELD et coll., Am. J. Physiol., 240, G255-266 (1981) ; BEINFELD et coll., Neuropeptides, 3, 411-427 (1983)).

Les composés de formule (I) présentent une toxicité faible. Leur $DL_{50}$ est généralement supérieure à 40 mg/kg par voie sous-cutanée chez la souris.

Les composés préférés sont ceux pour lesquels $R_1$ représente un radical tert-butyle.

D'un intérêt particulier, sont les composés suivants :

- {[(méthyl-3 phényl)-3 uréido]-3 phényl-3 N-phényl-propionamido}-2 acétate de tert-butyl-(RS).
- [(méthyl-3 phényl)-3 uréido]-3 N-(tert-butoxycarbonylméthyl) N-phényl-succinamate de méthyle-(RS).
- acide [(méthyl-3 phényl)-3 uréido]-3 N-(tert-butoxycarbonylméthyl) N-phényl-succinamique-(RS).
- [(méthyl-3 phényl)-3 uréido]-2 N-(tert-butoxycarbonylméthyl) N-phényl-succinamide-(RS).
- [(méthyl-3 phényl)-3 uréido]-4 N-(tert-butoxycarbonylméthyl) N-phényl-glutaramate de méthyle-(RS).
- acide [(méthyl-3 phényl)-3 uréido]-4 N-(tert-butoxy-carbonylméthyl) N-phényl-glutaramique-(RS).

Les exemples suivants illustrent l'invention sans la limiter.

EXEMPLE 1

Une suspension de 4,1 g d'anilino acétate de tert-butyle et de 5,97 g d'acide[(méthyl-3 phényl)-3 uréido]-3 phényl-3 propionique-(R,S) dans 50 $cm^3$ de dichloro-1,2 éthane anhydre est chauffée à reflux. On ajoute alors 1,45 $cm^3$ de chlorure de thionyle en maintenant le reflux jusqu'à la fin du dégagement gazeux. Le mélange réactionnel est alors versé dans 30 $cm^3$ d'une solution aqueuse saturée d'hydrogénocarbonate de sodium puis on ajoute 50 $cm^3$ de chlorure de méthylène. La phase organique est lavée par 50 $cm^3$ d'eau, séchée sur sulfate de magnésium et concentrée à sec sous pression réduite (2,7 kPa) à 40°C. Après deux recristallisations successives d'abord dans un mélange d'oxyde de diéthyle et d'oxyde de diisopropyle (50-50 en volumes) puis dans l'oxyde de diéthyle, on obtient 5,4 g de {[(méthyl-3 phényl)-3 uréido]-3 phényl-3 N-phényl- propionamido}-2 acétate de tert-butyle-(RS).

L'acide [(méthyl-3 phényl)-3 uréido]-3 phényl-3 propionique-(RS) peut être préparé de la manière suivante : à une suspension de 6,6 g d'acide amino-3 phényl-3 propionique-(RS) et de 3,36 g d'hydrogénocarbonate de sodium dans 100 $cm^3$ d'eau, on ajoute en 1 minute, 5,14 $cm^3$ d'isocyanate de méthyl-3 phényle. Le mélange est agité pendant 20 heures à une température voisine de 20°C puis est extrais par 3 fois 75 $cm^3$ d'acétate d'éthyle. La phase organique est lavée par 2 fois 50 $cm^3$ d'eau, les extraits aqueux sont réunis et acidifiés jusqu'à pH 1 par une solution aqueuse 4N d'acide chlorhydrique. Le précipité est filtré, lavé à l'eau et séché sous pression réduite (0,07 kPa) à 40°C. On obtient ainsi 8,7 g d'acide [(méthyl-3 phényl)-3 uréido]-3 phényl-3 propionique-(RS) fondant à 164°C.

EXEMPLE 2

A une suspension de 7,5 g d'acide [(méthyl-3 phényl)-3 uréido]-2 méthoxycarbonyl-3 propionique-(RS) et de 5,55 g de N-phényl-glycinate de tert-butyle dans 500 $cm^3$ de dichloro-1,2 éthane au reflux, on ajoute 3,2 g de chlorure de thionyle. Le milieu réactionnel est encore agité pendant 15 minutes au reflux, puis refroidi à 50°C et versé dans 200 $cm^3$ d'une solution aqueuse de bicarbonate de sodium à 10 %. La phase organique est décantée, lavée par 150 $cm^3$ d'eau, séchée sur sulfate de magnésium puis concentrée sous pression réduite (2,7 kPa). Le résidu huileux est purifié par chromatographie sur 120 g de silice (0,063-0,200 mm) contenus dans une colonne de 2,4 cm de diamètre [éluant : chlorure de méthylène-méthanol (99,5-0,5 en volumes)] en recueillant des fractions de 40 $cm^3$. Les fractions 20 à 56 sont réunies et concentrées à sec sous pression réduite (2,7 kPa) à 50°C. On obtient, après cristallisation dans l'oxyde de diéthyle, 2,6 g de [(méthyl-3 phényl)-3 uréido]-3 N-(tert-butoxycarbonyl- méthyl) N-phényl-succinamate de méthyle-(RS) fondant à 149°C.

L'acide [(méthyl-3 phényl)-3 uréido]-2 méthoxycarbonyl-3 proprionique-(RS) peut être préparé de la manière suivante : à une solution de 16,1 g d'acide amino-2 méthoxycarbonyl-3 propionique-(RS) et de 8,4 g de bicarbonate de sodium dans 160 $cm^3$ d'eau, on ajoute 13,3 g d'isocyanate de méthyl-3 phényle en 30 minutes à 17°C. Le milieu réactionnel est encore agité pendant 16 heures puis l'insoluble est filtré. Le filtrat est amené à pH 1 par de l'acide chlorhydrique 4N et extrait par trois fois 15 $cm^3$ d'acétate d'éthyle. Les extraits sont réunis, lavés par deux fois 15 $cm^3$ d'eau, séchés sur sulfate de magnésium puis amenés à sec sous pression réduite (2,7 kPa) à 70°C. Après cristallisation dans l'éther de pétrole, on obtient 24 g d'acide [(méthyl-3 phényl)-3 uréido]-2 méthoxycarbonyl-3 proprionique-(RS).

L'acide amino-2 méthoxycarbonyl-3 propionique-(RS) peut être prépare selon la méthode décrite par D. COLEMAN, J. Chem. Soc., 2294 (1951).

EXEMPLE 3

A une solution de 3,5 g de [(méthyl-3 phényl)-3 uréido]-3 N-(tert-butoxycarbonylméthyl) N-phényl-succinamate de méthyle-(RS) dans 120 $cm^3$ de méthanol, on ajoute 7,5 $cm^3$ de soude normale. Le milieu réactionnel est agité 24 heures à une température voisine de 20°C, puis on évapore le méthanol sous pression réduite (2,7 kPa) à 60°C. Le résidu est dilué par 210 $cm^3$ de soude 0,005 N. La phase aqueuse est lavée par deux fois 70 $cm^3$ d'acétate d'éthyle, amenée à pH acide par de l'acide chlorhydrique 4N et extraite par trois fois 80 $cm^3$ d'acétate d'éthyle. Les extraits organiques sont réunis, séchés sur sulfate de magnésium et amenés à sec sous pression réduite 2,7 kPa) à 70°C. Après cristallisation dans l'oxyde de diéthyle, on obtient 1,6 g d'acide [(méthyl-3 phényl)-3 uréido]-3 N-(tert-butoxycarbonylméthyl) N-phényl-succinamique-(RS) fondant à 198°C.

EXEMPLE 4

Dans une solution de 4,7 g de [(méthyl-3 phényl)-3 uréido]-3 N-(tert-butoxycarbonylméthyl) N-phényl-succinamate de méthyle-(RS) dans 150 $cm^3$ de méthanol, on fait passer un courant d'ammoniac pendant 11 heures à une température voisine de 25°C. La solution est ensuite dégazée par un courant d'azote et concentrée sous vide (2,7 kPa) à 60°C. Le résidu est cristallisé dans l'oxyde de diéthyle et le solide extrait par 40 $cm^3$ de dichlorométhane. La solution ainsi obtenue est purifiée par chromatographie sur 25 g de silice (0,063-0,200 mm) contenus dans une colonne de 1,6 cm de diamètre [éluant : 1100 $cm^3$ de chlorure de méthylène puis chlorure de méthylène-méthanol (98,5-1,5 en volumes)] en recueillant des fractions de 15 $cm^3$. Les fractions 80 à 127 sont réunies et concentrées à sec sous pression réduite (2,7 kPa) à 60°C. On obtient, après cristallisation dans l'éther de pétrole 1,1 g de [(méthyl-3 phényl)-3 uréido]-2 N-(tert-butoxycarbonyl- méthyl) N-phényl-succinamide-(RS) fondant à 192°C.

EXEMPLE 5

On opère d'une manière analogue à celle décrite à l'exemple 2, mais à partir de 1,47 g d'acide [-(méthyl-3 phényl)-3 uréido]-2 méthoxycarbonyl-4 butyrique-(RS), de 1,04 g de N-phényl-glycinate de tert-butyle et de 0,60 g de chlorure de thionyle. Le produit obtenu est purifié par chromatographie sur 50 g de silice (0,063-0,200 mm) contenus dans une colonne de 1,8 cm de diamètre [éluant : 225 $cm^3$ de chlorure de méthylène puis chlorure de méthylène-méthanol (99-1 en volumes)] en recueillant des fractions de 15 $cm^3$. Les fractions 36 à 40 sont réunies et concentrées à sec sous pression réduite (2,7 kPa) à 50°C. On obtient, après cristallisation dans l'oxyde de diéthyle puis recristallisation dans un mélange d'hexane et d'éthanol (85-15 en volumes), 1,9 g de [(méthyl-3 phényl)-3 uréido]-4 N-(tert-butoxycarbonylméthyl) N-phényl-glutaramate de méthyl-(RS) fondant à 139°C.

L'acide [(méthyl-3 phényl-3 uréido]-2 méthoxycarbonyl-4 butyrique-(RS) peut être préparé d'une manière analogue à celle décrite à l'exemple 2 pour la préparation de l'acide [(méthyl-3 phényl)-3 uréido]-2 méthoxycarbonyl-3 propionique-(RS), mais à partir de 16,1 g d'acide amino-2 méthoxycarbonyl-4 butyrique-(RS) et de 13,3 g d'isocyanate de méthyl-3 phényle. On obtient ainsi 23,5 g d'acide [(méthyl-3 phényl)-3 uréido]-2 méthoxycarbonyl-4 butyrique-(RS) fondant à 127°C.

EXEMPLE 6

On opère comme à l'exemple 3, mais à partir de 4,7 g de [(méthyl-3 phényl)-3 uréido]-4 N-(tert-butoxycarbonylméthyl) N-phényl-glutaramate de méthyle-(RS) et de 9,7 $cm^3$ de soude normale. Le produit obtenu est purifié par chromatographie sur 60 g de silice (0,063-0,200 mm) contenus dans une colonne de 2,4 cm de diamètre [éluant : 1000 $cm^3$ de chlorure de méthylène- méthanol (99-1 en volumes) puis chlorure de méthylène-méthanol (97-3 en volumes)] en recueillant des fractions de 25 $cm^3$. Les fractions 42 à 105 sont réunies et concentrées à sec sous pression réduite (2,7 kPa) à 60°C. On obtient, après cristallisation dans l'oxyde de diéthyle 1,45 g d'acide [(méthyl-3 phényl)-3 uréido]-4 N-(tert-butoxycarbonylméthyl) N-phényl-glutaramique-(RS) fondant à 191°C.

La présente invention concerne également les médicaments constitués par au moins un composés de formule (I) à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme composition liquides pour administration orale, on peut utiliser des solutions, des suspensions, des émulsions, des sirops, des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, des pommades, des lotions, des collyres, des collutoires, des gouttes nasales ou des aérosols.

En thérapeutique humaine, les composés selon l'invention sont particulièrement utiles dans le traitement et la prévention des désordres liés à la CCK et à la gastrine au niveau du système nerveux et de

l'appareil gastrointestinal. Ces composés peuvent donc être utilisés dans le traitement et la prévention des psychoses, des troubles anxieux, de la maladie de Parkinson, de la diskinésie tardive, du syndrome du colon irritable, de la pancréatite aiguë, des ulcères, des désordres de la motilité intestinale et de certaines tumeurs de l'oesophage inférieur, du colon et de l'intestin, comme potentialisateur de l'activité analgésique des médicaments analgésiques narcotiques et non narcotiques et comme régulateur de l'appétit.

Les doses dépendent de l'effect recherché, de la durée du traitement et de la voie d'administration utilisée ; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

EXEMPLE A

On prépare, selon la technique habituelle, des gélules dosées à 50 mg de produit actif ayant la composition suivante :

| | |
|---|---|
| - {[(méthyl-3 phényl)-3 uréido]-3 phényl-3 N-phényl propionamido}-2 acétate de tert-butyle-(RS) | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

EXEMPLE B

On prépare, selon la technique habituelle, des comprimés dosés à 50 mg de produit actif ayant la composition suivante :

| | |
|---|---|
| - {[(méthyl-3 phényl)-3 uréido]-3 N-(tert-butoxycarbonylméthyl) N-phényl-succinamate de méthyle-(RS) | 50 mg |
| - Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| - Silice colloïdale | 2 mg |
| - Mélange d'hydroxyméthylcellulose, glycérine, oxyde de titane (72-3, 5-24, 5) | q.s.p. 1 comprimé pelliculé terminé à 245 mg |

EXEMPLE C

On prépare une solution injectable contenant 10 mg de produit actif ayant la composition suivante :

| | |
|---|---|
| - acide [(méthyl-3 phényl)-3 uréido]-3 N-(tert-butoxycarbonylméthyl) N-phényl-succinamique-(RS) | 10 mg |
| - Acide benzoïque | 80 mg |
| - Alcool benzylique | 0,06 $cm^3$ |
| - Benzoate de sodium | 80 mg |
| - Ethanol à 95 % | 0,4 $cm^3$ |
| - Hydroxyde de sodium | 24 mg |
| - Propylène glycol | 1,6 $cm^3$ |
| - eau | q.s.p. 4 $cm^3$ |

**Revendications**

**Revendications pour les Etats contractants suivants : AT, BE, CH, DE, DK, FR, GB, IT, LI, LU, NL, SE**

**1.** Composés de formule :

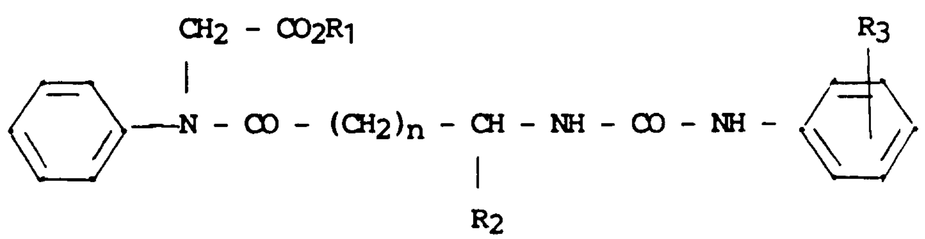

(I)

dans laquelle :

- $R_1$ représente un radical alkyle,
- $R_2$ représente un radical phényle ou une chaîne $-(CH_2)_m-CO-R_4$ dans laquelle m est égal à 0, 1 ou 2 et $R_4$ représente un radical hydroxy, alcoxy ou amino,
- $R_3$ représente un atome d'hydrogène ou d'halogène ou un radical alkyle, alcoxy ou alkythio,
- n est égal à 0 ou 1,

étant entendu que les radicaux alkyle et alcoxy et les portions alkyle contiennent 1 à 4 atomes de carbone en chaîne droite ou ramifiée, ainsi que leurs racémiques et énantiomères et leurs sels.

**2.** Composés selon la revendication 1 pour lesquels $R_1$ représente un radical tert-butyle.

**3.** Composés selon l'une des revendications 1 ou 2 pour lesquels les atomes d'halogène sont les atomes de chlore, brome ou fluor.

**4.** Procédé de préparation des composés de formule (I) selon la revendication 1 pour lesquels $R_2$ représente un radical phényle ou une chaîne $-(CH_2)_m-CO-R_4$ dans laquelle m est égal à 0, 1 ou 2 et $R_4$ représente un radical alcoxy caractérisé en ce que l'on fait réagir une amine de formule :

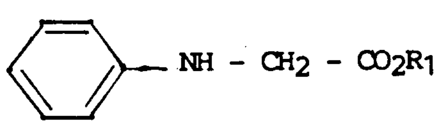

(II)

dans laquelle $R_1$ a les mêmes significations que dans la revendication 1, sur un acide de formule :

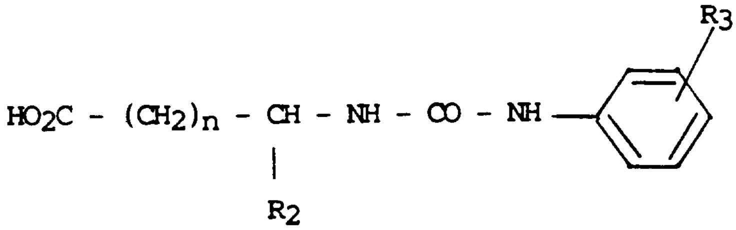

(III)

dans laquelle n et $R_3$ ont les mêmes significations que dans la revendication 1 et $R_2$ a les mêmes significations que précédemment, ou un dérivé réactif de cet acide puis isole le produit.

**5.** Procédé de préparation des composés de formule (I) selon la revendication 1 pour lesquels $R_2$ représente une chaîne $-(CH_2)_m-CO-R_4$ dans laquelle m est égal à 0, 1 ou 2 et $R_4$ représente un radical hydroxy caractérisé en ce que l'on hydrolyse les composés de formule (I) correspondants pour lesquels $R_4$ représente un radical alcoxy puis isole le produit.

**6.** Procédé de préparation des composés de formule (I) selon la revendication 1 pour lesquels $R_2$ représente une chaîne $-(CH_2)_m-CO-R_4$ dans laquelle m est égal à 0,1 ou 2 et $R_4$ représente un radical amino caractérisé en ce que l'on fait réagir l'ammoniac sur un composé de formule (I) correspondant pour lequel $R_4$ représente un radical alcoxy puis isole le produit.

**7.** Médicaments caractérisés en ce qu'ils contiennent en tant que principe actif au moins un composé de formule (I) selon la revendication 1.

**8.** Médicaments selon la revendication 7 pour le traitement ou la prévention des désordres liés à la CCK et à la gastrine au niveau du système nerveux et de l'appareil gastrointestinal.

**Revendications pour les Etats contractants suivants : ES, GR**

**1.** Procédé de préparation des composés de formule :

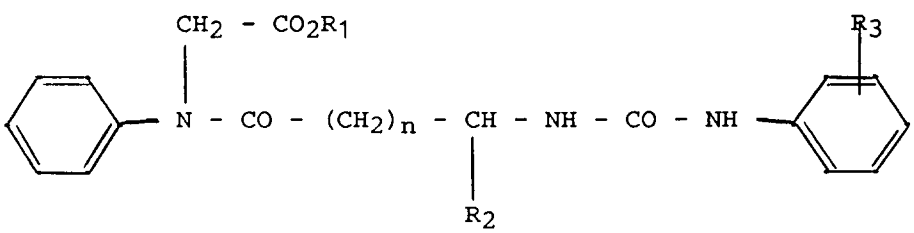

(I)

dans laquelle :

- $R_1$ représente un radical alkyle,
- $R_2$ représente un radical phényle ou une chaîne $-(CH_2)_m-CO-R_4$ dans laquelle m est égal à 0, 1 ou 2 et $R_4$ représente un radical hydroxy, alcoxy ou amino,
- $R_3$ représente un atome d'hydrogène ou d'halogène ou un radical alkyle, alcoxy ou alkylthio,
- n est égal à 0 ou 1,

étant entendu que les radicaux alkyle et alcoxy et les portions alkyle contiennent 1 à 4 atomes de carbone en chaîne droite ou ramifiée, ainsi que leurs racémiques et énantiomères,

caractérisé en ce que :

A - pour la préparation des composés pour lesquels $R_2$ représente un radical phényle ou une chaîne $-(CH_2)_m-CO-R_4$ dans laquelle m est égal à 0, 1 ou 2 et $R_4$ représente un radical alcoxy, on fait réagir une amine de formule :

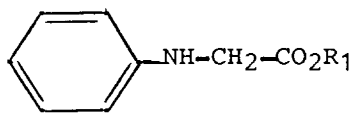

(II)

dans laquelle $R_1$ a les mêmes significations que dans la formule (I), sur un acide de formule :

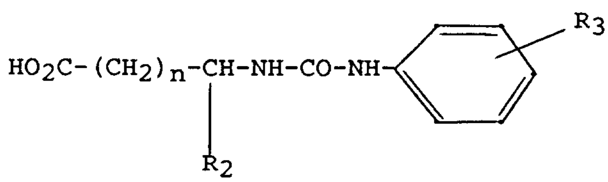

(III)

dans laquelle n et $R_3$ ont les mêmes significations que dans la formule (I) et $R_2$ a les mêmes significations que précédemment, ou un dérivé réactif de cet acide puis isole le produit,
B - pour la préparation des composés pour lesquels $R_2$ représente une chaîne $-(CH_2)_m-CO-R_4$ dans laquelle m est égal à 0, 1, ou 2 et $R_4$ représente un radical hydroxy, on hydrolyse les composés de formule (I) correspondants pour lesquels $R_4$ représente un radical alcoxy puis isole le produit,
C - pour la préparation des composés pour lesquels $R_2$ représente une chaîne $-(CH_2)_m-CO-R_4$ dans laquelle m est égal à 0, 1 ou 2 et $R_4$ représente un radical amino, on fait réagir l'ammoniac sur un composé de formule (I) correspondant pour lequel $R_4$ représente un radical alcoxy puis isole le produit.

**2.** Procédé selon la revendication 1 pour la préparation des composés pour lesquels $R_1$ représente un radical tert-butyle.

**3.** Procédé selon la revendication 1 ou 2 pour la préparation des composés pour lesquels les atomes d'halogène sont des atomes de chlore, brome ou fluor.

**Claims**
**Claims for the following Contracting States : AT, BE, CH, DE, DK, FR, GB, IT, LI, NL, SE**

**1.** Compounds of formula

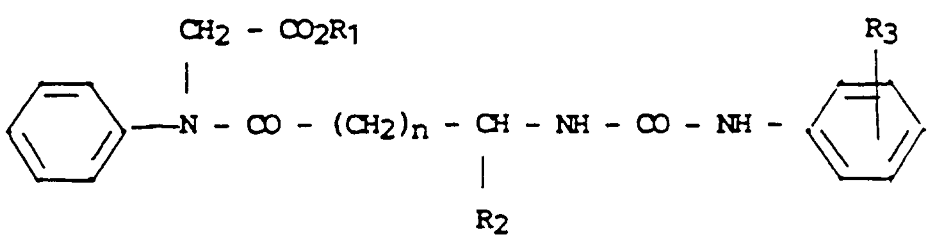

(I)

in which:
- $R_1$ represents an alkyl radical,
- $R_2$ represents a phenyl radical or a chain $-(CH_2)_m-CO-R_4$ in which m is equal to 0, 1 or 2 and $R_4$ represents a hydroxyl, alkoxy or amino radical,
- $R_3$ represents a hydrogen or halogen atom or an alkyl, alkoxy or alkylthio radical, and
- n is equal to 0 or 1,

on the understanding that the alkyl and alkoxy radicals and alkyl portions contain 1 to 4 carbon atoms in a straight or branched chain, as well as their racemates and enantiomers and their salts.

**2.** Compounds according to claim 1 for which $R_1$ represents a tert-butyl radical.

3. Compounds according to one of claims 1 and 2 for which the halogen atoms are chlorine, bromine or fluorine atoms.

4. Process for preparing the compounds of formula (I) according to claim 1 for which $R_2$ represents a phenyl radical or a chain $-(CH_2)_m-CO-R_4$ in which m is equal to 0, 1 or 2 and $R_4$ represents an alkoxy radical, characterized in that an amine of formula

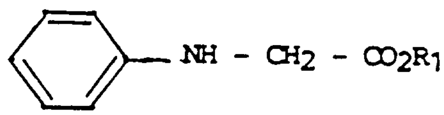 (II)

in which $R_1$ has the same meanings as in claim 1, is reacted with an acid of formula:

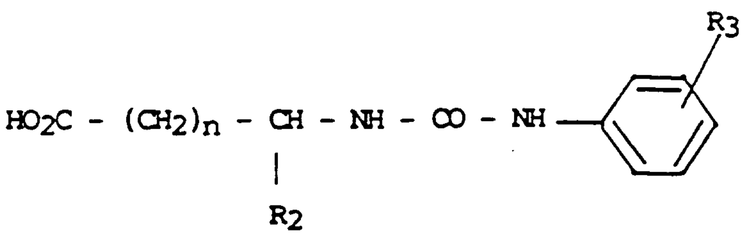 (III)

in which n and $R_3$ have the same meanings as in claim 1 and $R_2$ has the same meanings as above, or a reactive derivative of this acid, and the product is then isolated.

5. Process for preparing the compounds of formula (I) according to claim 1 for which $R_2$ represents a chain $-(CH_2)_m-CO-R_4$ in which m is equal to 0, 1 or 2 and $R_4$ represents a hydroxyl radical, characterized in that the corresponding compound of formula (I) in which $R_4$ represents an alkoxy radical is hydrolysed and the product is then isolated.

6. Process for preparing the compounds of formula (I) according to claim 1 for which $R_2$ represents a chain $-(CH_2)_m-CO-R_4$ in which m is equal to 0, 1 or 2 and $R_4$ represents an amino radical, characterized in that ammonia is reacted with a corresponding compound of formula (I) for which $R_4$ represents an alkoxy radical and the product is then isolated.

7. Medicinal products, characterized in that they contain as active principle at least one compound of formula (I) according to claim 1.

8. Medicinal products according to claim 7, for the treatment or prevention of disorders associated with CCK and gastrin affecting the nervous system and the gastro-intestinal system.

**Claims for the following Contracting States : ES, GR**

1. Process for preparing the compounds of formula

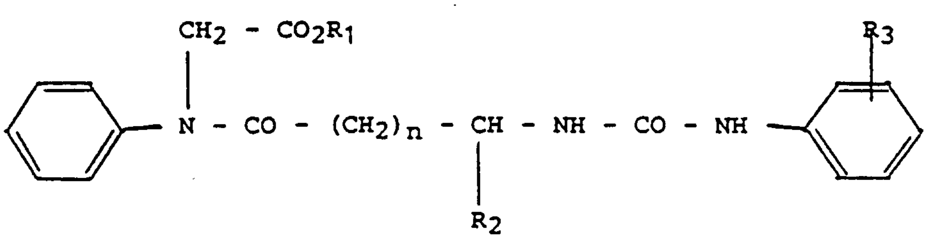 (I)

in which:

- $R_1$ represents an alkyl radical,
- $R_2$ represents a phenyl radical or a chain $-(CH_2)_m-CO-R_4$ in which m is equal to 0, 1 or 2 and $R_4$ represents a hydroxyl, alkoxy or amino radical,
- $R_3$ represents a hydrogen or halogen atom or an alkyl, alkoxy or alkylthio radical, and
- n is equal to 0 or 1,

on the understanding that the alkyl and alkoxy radicals and alkyl portions contain 1 to 4 carbon atoms in a straight or branched chain, as well as their racemates and enantiomers,
characterized in that:

A - for the preparation of the compounds for which $R_2$ represents a phenyl radical or a chain $-(CH_2)_m-CO-R_4$ in which m is equal to 0, 1 or 2 and $R_4$ represents an alkoxy radical, an amine of formula

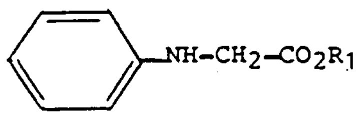

(II)

in which $R_1$ has the same meanings as in the formula (I), is reacted with an acid of formula:

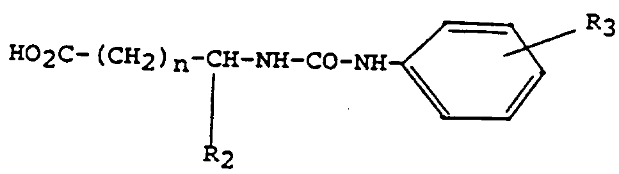

(III)

in which n and $R_3$ have the same meanings as in the formula (I) and $R_2$ has the same meanings as above, or a reactive derivative of this acid, and the product is then isolated,

B - for the preparation of the compounds for which $R_2$ represents a chain $-(CH_2)_m-CO-R_4$ in which m is equal to 0, 1 or 2 and $R_4$ represents a hydroxyl radical, the corresponding compound of formula (I) in which $R_4$ represents an alkoxy radical is hydrolysed and the product is then isolated,

C - for the preparation of the compound for which $R_2$ represents a chain $-(CH_2)_m-CO-R_4$ in which m is equal to 0, 1 or 2 and $R_4$ represents an amino radical, ammonia is reacted with a corresponding compound of formula (I) for which $R_4$ represents an alkoxy radical and the product is then isolated.

2. Process according to claim 1, for the preparation of the compounds for which $R_1$ represents a tert-butyl radical.

3. Process according to claim 1 or 2, for the preparation of the compounds for which the halogen atoms are chlorine, bromine or fluorine atoms.

**Patentansprüche**
**Patentansprüche für folgende Vertragsstaaten : AT, BE, CH, DE, DK, FR, GB, IT, LI, LU, NL, SE**

1. Verbindungen mit der Formel

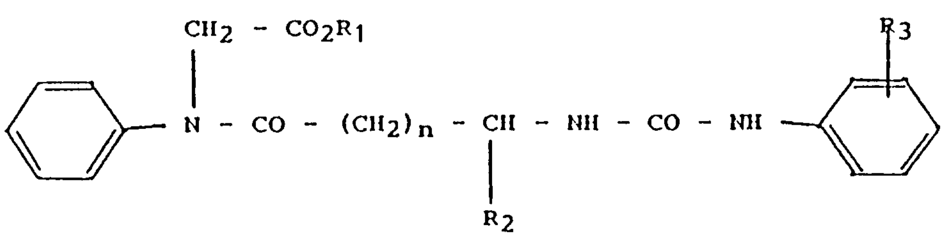

(I)

in der

- $R_1$ für einen Alkylrest steht,

- $R_2$ für einen Phenylrest oder eine Kette aus $-(CH_2)_m-CO-R_4$ steht, in der m gleich 0, 1 oder 2 ist, und $R_4$ für einen Hydroxyl-, Alkoxy- oder Aminorest steht,
- $R_3$ für ein Wasserstoff- oder Halogenatom oder für einen Alkyl-, Alkoxy- oder Alkylthiorest steht,
- n gleich 0 oder 1 ist,

wobei die Alkyl- und Alkoxyreste und die Alkylantelle 1 bis 4 Kohlenstoffatome in geradliniger oder verzweigter Kette enthalten, sowie ihre Racemate und Enantiomere und ihre Salze.

**2.** Verbindungen gemäß Anspruch 1, in denen $R_1$ für einen tert-Butylrest steht.

**3.** Verbindungen gemäß einem der Ansprüche 1 oder 2, in denen die Halogenatome Chlor-, Brom- oder Fluoratome sind.

**4.** Verfahren zur Herstellung der Verbindungen mit der Formel (I) gemäß Anspruch 1, in denen $R_2$ für einen Phenylrest oder eine Kette aus $-(CH_2)_m-CO-R_4$ steht, in der m gleich 0, 1 oder 2 ist, und $R_4$ für einen Alkoxyrest steht, dadurch gekennzeichnet, daß man ein Amin mit der Formel:

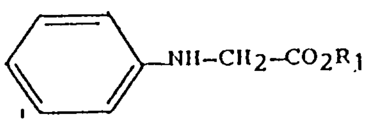

(II)

in der $R_1$ die gleichen Bedeutungen hat wie in Anspruch 1, mit einer Säure mit folgender Formel reagieren läßt:

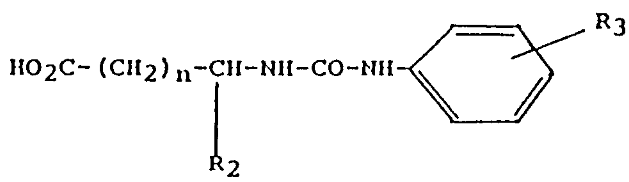

(III)

in der n und $R_3$ die gleichen Bedeutungen wie in Anspruch 1 haben und $R_2$ die gleichen Bedeutungen wie vorher, oder mit einem reaktiven Derivat dieser Säure, daß man dann das Produkt isoliert.

**5.** Verfahren zur Herstellung der Verbindungen nach Formel (I) gemäß Anspruch 1, in denen $R_2$ für eine Kette aus $-(CH_2)_m-CO-R_4$ steht, in der m gleich 0, 1 oder 2 ist, und $R_4$ für einen Hydroxylrest steht, dadurch gekennzeichnet, daß man die entsprechenden Verbindungen mit der Formel (I) hydrolysiert, in denen $R_4$ für einen Alkoxyrest sicht, daß man dann das Produkt isoliert.

**6.** Verfahren zur Herstellung der Verbindungen mit der Formel (I) gemäß Anspruch 1, in denen $R_2$ für eine Kette aus $-(CH_2)_m-CO-R_4$ steht, in der m gleich 0, 1 oder 2 ist, und $R_4$ für einen Aminorest steht, dadurch gekennzeichnet, daß man Ammoniak mit einer entsprechenden Verbindung der Formel (I) reagieren läßt, in der $R_4$ für einen Alkoxyrest steht, daß man dann das Produkt isoliert.

**7.** Arzneimittel, dadurch gekennzeichnet, daß sie als Wirkstoff wenigstens eine Verbindung mit der Formel (I) gemäß Anspruch 1 enthalten.

**8.** Arzneimittel gemäß Anspruch 7 zur Behandlung oder der Prävention von Störungen im Nervensystem und dem Gastrointestinaltrakt, die mit der CCK und dem Gastrin in Verbindung stehen.

**Patentansprüche für folgende Verstragsstaaten : ES, GR**

1. Verfahren zur Herstellung der Verbindungen mit der Formel:

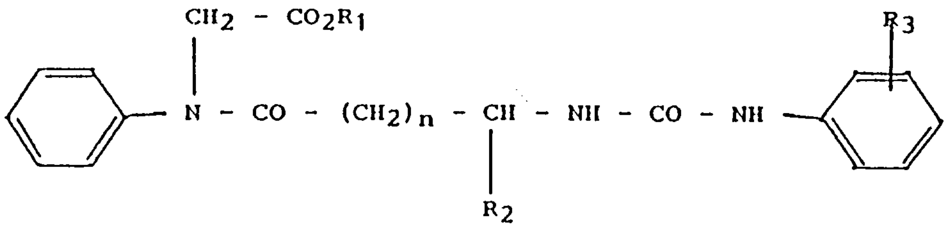

(I)

in der
- $R_1$ für einen Alkylrest steht,
- $R_2$ für einen Phenylrest oder eine Kette aus $-(CH_2)_m-CO-R_4$ steht, in der m gleich 0, 1 oder 2 ist, und $R_4$ für einen Hydroxyl- Alkoxy- oder Aminorest steht,
- $R_3$ für ein Wasserstoff- oder Halogenatom oder für einen Alkyl-, Alkoxy- oder Alkylthiorest steht,
- n gleich 0 oder 1 ist,

wobei die Alkyl- und Alkoxyreste und die Alkylanteile 1 bis 4 Kohlenstoffatome in geradliniger oder verzweigter Kette enthalten, sowie ihre Racemate und Enantiomere, dadurch gekennzeichnet, daß man

A - für die Herstellung der Verbindungen, in denen $R_2$ für einen Phenylrest oder eine Kette aus $-(CH_2)_m-CO-R_4$ steht, in der m gleich 0, 1 oder 2 ist, und $R_4$ für einen Alkoxyrest steht, ein Amin mit der Formel:

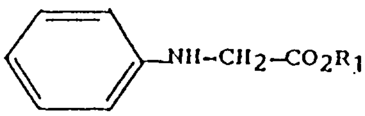

(II)

in der $R_1$ die gleichen Bedeutungen hat wie in der Formel (I), mit einer Säure mit folgender Formel reagieren läßt:

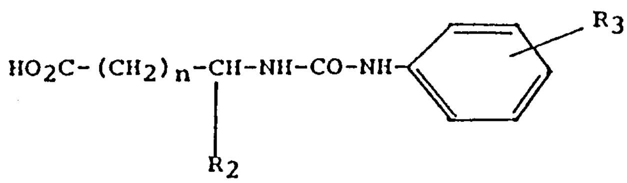

(III)

in der n und $R_3$ die gleichen Bedeutungen haben wie in der Formel (I) und $R_2$ die gleichen Bedeutungen hat wie vorher, oder mit einem reaktiven Derivat dieser Säure, daß man dann dieses Produkt isoliert,

B - für die Herstellung der Verbindungen, in denen $R_2$ für eine Kette aus $-(CH_2)_m-CO-R_4$ steht, in der m gleich 0, 1 oder 2 ist, und $R_4$ für einen Hydroxylrest steht, die entsprechenden Verbindungen mit der Formel (I) hydrolysiert, in denen $R_4$ für einen Alkoxyrest steht, daß man dann das Produkt isoliert.

C - für die Herstellung der Verbindungen, in denen $R_2$ für eine Kette aus $-(CH_2)_m-CO-R_4$ steht, in der m gleich 0, 1 oder 2 ist, und $R_4$ für einen Aminorest steht, Ammoniak mit einer entsprechenden Verbindung mit der Formel (I) reagieren läßt, in der $R_4$ für einen Alkoxyrest steht, daß man dann das Produkt isoliert.

2. Verfahren gemäß Anspruch 1 für die Herstellung der Verbindungen, in denen $R_1$ für einen tert-Butylrest steht.

**3.** Verfahren gemäß Anspruch 1 oder 2 für die Herstellung der Verbindungen. in denen die Halogenatome Chlor-, Brom- oder Fluoratome sind.